# EUROPÄISCHE PATENTSCHRIFT

(11) **EP 3 459 489 B1**
(45) Veröffentlichungstag und Bekanntmachung des Hinweises auf die Patenterteilung: **10.03.2021**
(21) Anmeldenummer: 18194731.8
(22) Anmeldetag: 17.09.2018
(51) Int. Cl.: A61B 50/20, A61B 50/30, B65D 43/16, A45D 40/22

(54) **SCHUTZKAPPE MIT DECKEL UND SPRINGÖFFNUNG**
PROTECTION CAP WITH A COVER AND SPRING OPENING
CAPOT DE PROTECTION POURVU DE COUVERCLE ET D'OUVERTURE À RESSORT

(30) Priorität: 26.09.2017 DE 102017122259
(43) Veröffentlichungstag der Anmeldung: 27.03.2019
(73) Patentinhaber: Rösler IP GmbH, 88138 Hergensweiler (DE)
(72) Erfinder: RÖSLER, Thiemo, 88239 Wangen (DE)
(74) Vertreter: Riebling, Peter

(56) Entgegenhaltungen:
- WO-A1-03/084835
- DE-A1-102014 001 845
- US-A1- 2004 159 661
- US-A1- 2013 175 271

## Beschreibung

Die Erfindung betrifft eine Schutzkappe mit Springöffnung nach dem Oberbegriff des Patentanspruchs 1.

Ein Behälter mit Springöffnung ist beispielsweise mit dem Gegenstand der DE602005004804T2 (EP1799577B1) bekannt geworden. Der dortige Behälter zeichnet sich dadurch aus, dass die Gelenkanordnung zwischen einem Deckel und einem Behälter mit einem Elastomerfederteil ausgestattet ist. Somit kann der Deckel bei Betätigung einer Verschlusseinrichtung im Bereich seiner Schwenkachse selbsttätig durch die Kraft des Elastomerfederteils aufspringen, wodurch demnach ein Behälter mit einem Deckel mit Springöffnung geschaffen ist.

Kennzeichnend für diese Konstruktion ist, dass eine Schwenkachse mit einem Elastomerfederteil an der Außenseite des Deckels angeordnet ist und in der geschlossenen Position des Deckels dieser unter Federkraft in Öffnungsrichtung vorgespannt ist.

Bei dieser bekannten Konstruktion liegt die Kraftangriffsstelle des Elastomerfederteils am Deckel. Zu diesem Zweck ist die Schwenkachse des Gelenks über die Behälteraußenwand hinaus nach außen verlagert.

Durch die beabstandete Anordnung des Elastomerfederteils zur Schwenkachse zwischen dem Deckel und dem Behälterunterteil wird das Elastomerfederteil beim Schließen des Deckels gespannt, sodass bei Betätigung der Verschlusseinrichtung der Deckel selbsttätig aufspringt. Dies wird als Springöffnung des Deckels bezeichnet.

Die bekannte Gelenkanordnung an der Außenseite des Behälters hat sich im großen Umfang bewährt, hat aber den Nachteil, dass ein hoher konstruktiver Aufwand für die Schaffung einer solchen Gelenkanordnung notwendig ist.

Die bekannte Gelenkanordnung besteht aus einem Gelenkstift, der von einer Gelenklagerung umgriffen ist, was mit einem hohen konstruktiven Herstellungsaufwand verbunden ist.

Auch die Anordnung des Elastomerfederteils, welches als einfaches, planes und bandförmiges Elastomerband ausgebildet ist, ist aufwendig, denn es muss zwischen zwei außerhalb der Behälteranordnung angeordnete, behälterseitige Schenkel über geeignete Anbindungsstellen verbunden werden.

Damit besteht ein Nachteil, dass durch die externe Anordnung der Schenkel an den Außenwänden des Behälters, eine Bruchgefahr für die Schenkel besteht, die ungeschützt sind. Fällt ein solcher Behälter zu Boden, dann besteht die Gefahr, dass die Schenkel abbrechen.

Weiterhin ist der konstruktive Aufwand bei der Schaffung von Verbindungsstellen an den von den Behälterwänden abragenden Schenkel bezüglich der Befestigung des Elastomerfederbandes aufwendig und betriebsunsicher.

Die Federkraft einer solchen Springfederanordnung ist außerdem eingeschränkt, weil das Elastomerfederband nur eine kurze Spannlänge hat und deshalb entweder kein hohe Öffnungsspringkraft gegeben ist oder - bei hohen Spannkräften - die Gefahr besteht, dass das Springfederband einreißt oder versagt.

Aufgrund der bandförmigen Ausbildung des Elastomerfederteils besteht demnach der Nachteil, dass sämtliche Biegevorgänge nur über das plane, im Querschnitt rechteckförmige Elastomerband stattfinden, was mit einer verkürzten Lebensdauer verbunden sein könnte.

Mit dem Gegenstand der CH 619 413 A5 ist ein Flaschenverschluss für feste und deformierbare Behälter nach dem Oberbegriff des geltenden Anspruches 1 bekannt geworden, bei dem ein den Flaschenverschluss in Öffnungsrichtung vorspannender Springkörper vorgesehen ist, der werkstoffeinstückig mit der Flaschenwandung und dem Verschluss verbunden ist und ein etwa U-förmiges Profil aufweist. Ein solcher Flaschenverschluss ist nicht auf eine lange Lebensdauer mit einer Vielzahl von Lastwechselspielen ausgelegt.

Weil der Springkörper aus dem gleichen härteren Werkstoff wie der Werkstoff des Verschlusses besteht, ist dies mit dem Nachteil verbunden, dass eine hohe Spannkraft bei langer Lebensdauer nicht erreicht werden kann. Vielmehr besteht bei länger dauerndem Gebrauch Bruchgefahr für den Springkörper. Die Spannkraft soll nur so ausgelegt sein, dass der Flaschenverschluss genügend weit von der Flaschenöffnung abschwenkt, ohne dass eine definierte Offenstellung gefordert ist.

In der US 2013/175271 A1 und der WO 03/084835 A1 ist eine Schutzkappe mit den Merkmalen des Oberbegriffs des Patentanspruchs 1 offenbart.

DE 10 2014 001 845 A1 offenbart eine Schutzkappe mit Springöffnung bestehend aus einem Oberteil und einem Unterteil, die über Filmscharniere einseitig schwenkbar miteinander verbunden und im Schließzustand miteinander verrastet sind, wobei eine Rastvorrichtung zwischen dem Ober- und dem Unterteil an den Seitenwänden angeordnet ist.

Der Erfindung liegt deshalb die Aufgabe zugrunde, eine Schutzkappe nach dem Oberbegriff des Patentanspruchs 1 so weiterzubilden, dass sie zur Aufnahme eines Gegenstands geeignet ist und eine Springöffnung zwischen zwei einander gegenüberliegenden Teilen der Schutzkappe bei hoher Lebensdauer und hoher Spannkraft mit hoher Betriebssicherheit gegeben ist.

Zur Lösung der gestellten Aufgabe ist die Erfindung durch die technische Lehre des Anspruchs 1 gekennzeichnet.

Der Springkörper ist an beiden Seiten werkstoffeinstückig mit elastomeren Lappen versehen, und jeder Lappen durchsetzt einen Schlitz in der jeweiligen Stirnseite der Behälterwand und setzt sich dort in Form einer elastomeren Verbindungsbahn fort. Daraus ergibt sich der Vorteil, dass eine lange Spannlänge des Springkörpers gewährleistet ist, und dass der Springkörper eine hohe Anzahl von Belastungsspielen bei hoher Federkraft aushält und die Lebensdauer verlängert ist.

Der profilierte Springkörper im Profil entweder als U- oder ein vom U-Profil abweichendes Profil, wie zum Beispiel ein L- oder ein Dreiecks-Profil ausgebildet. Er ist in einer Weiterbildung bevorzugt auch wellenförmig ausgebildet, was bedeutet, dass das U-Profil in der Seitenansicht verdoppelt oder verdreifacht ist.

In einer bevorzugten Ausführung ist vorgesehen, dass der profilierte Springkörper nicht direkt an den Stirnseiten der aneinander zugeordneten Behälterteile angebunden ist, sondern sich in Form einer elastomeren Verbindungsbahn in den Innenraum der beiden Behälterteile hinein erstreckt.

Die Schutzkappe ist vorzugsweise für den Spitzenschutz von chirurgischen Instrumenten ausgebildet, die sich von dem Behälter nach dem Stand der Technik dadurch unterscheidet, dass die eine Stirnseite der Schutzkappe als Öffnungsseite ausgebildet ist, in welche zum Beispiel die Spitze eines zu schützenden chirurgischen Instrumentes oder ein anderer empfindlicher Gegenstand eingeschoben werden kann. Wohingegen bei einem Behälter die Öffnungsseite der Schutzkappe durch eine stirnseitige Behälterwand verschlossen ist.

Von Vorteil ist, wenn die Schutzkappe in ihrem Innenraum einander gegenüberliegende Lamellenpakete aufweist, wobei das eine Lamellenpaket an der Innenseite des Oberteils und das gegenüberliegende Lamellenpaket an der Innenseite des Unterteils angeordnet ist und die beiden Lamellenpakete zwischen sich eine Klemmaufnahme bilden, die den dort eingeschobenen, zu schützenden Gegenstand gegen ein Herausziehen schützt.

Derartige Lamellenpakete bestehen aus einzelnen, im gegenseitigen Abstand hintereinander angeordneten, elastomer biegbaren Lamellen, die beliebige Formgebung und Konturierung aufweisen können.

Sie können fluchtend einander gegenüberliegend angeordnet sein, sie können aber auch zueinander versetzt angeordnet sein, um bestimmte widerhakenartige Vorsprünge für einen dort einzuschiebenden Gegenstand zu bilden.

Statt der Ausbildung von Lamellenpaketen, die aus einzelnen parallel und im gegenseitigen Abstand zueinander angeordneten elastomeren Lamellen bestehen, können auch andere Aufnahme- oder Schutz- oder Klemmvorrichtungen verwendet werden, wie zum Beispiel elastomere Druckpolster, die zwischen sich ebenfalls eine Klemmaufnahme bilden.

Das eine Elastomerpolster ist dann an einer Innenseite des Oberteils und das andere Elastomerpolster an der Innenseite des Unterteils angeordnet und sie bilden somit zwischen sich eine Klemmaufnahme.

Die Betätigung der Rastvorrichtung ist nun nicht mehr an der (stirnseitigen) Öffnungsseite - in welche der zu schützende Gegenstand eingeschoben wird - angeordnet, sondern die Betätigung ist bevorzugt an den beiden aneinander gegenüberliegenden Seitenwänden zwischen dem Oberteil und dem Unterteil angeordnet und zwar seitlich benachbart der stirnseitigen Öffnungsseite.

Eine solche rastende Betätigung kann aus von den Behälterseitenwänden herausstehenden Handhaben bestehen, die bevorzugt rundprofiliert oder abgerundet ausgebildet sind. Wenn jeweils Fingerdruck mit zwei Fingern einer Hand gegen die einander gegenüberliegenden Bedienungshandhaben ausgeübt wird, federn die Handhaben unter Ausnutzung der elastomeren Federeigenschaften der Seitenwände des Unterteils nach innen und nehmen gleichzeitig die mit der jeweiligen Bedienungshandhabe verbundene Rastklinke mit, die damit außer Eingriff mit einer im Oberteil angeordneten Rastausnehmung gelangt.

Die Erfindung ist also nicht auf die spezielle Anordnung von gegenüberliegenden Bedienungshandhaben mit daran angeformten Rastklinken beschränkt, obwohl diese Ausführung besonders dann bevorzugt wird, wenn die erfindungsgemäße Schutzkappe mit der erfindungsgemäßen Springfunktion für den Schutz und die Aufnahme von empfindlichen Operationsinstrumenten, wie z.B. Skalpelle, Knochensägen oder Pinzetten verwendet wird.

Im Operationssaal kann die Schutzkappe mit den Fingern einer behandschuhten Hand ergriffen werden. Dabei werden die beiden Bedienungshandhaben gegeneinander gepresst, um so die damit verbundenen Rastklinken im Unterteil außer Eingriff mit den Rastausnehmungen im Oberteil zu bringen, sodass aufgrund der Federkraft des Springkörpers das Oberteil selbsttätig aufspringt und in dieser geöffneten Stellung verbleibt.

Durch die Verwendung eines profilierten Springkörpers ergibt sich eine wesentliche höhere Federkraft als vergleichsweise die Verwendung eines kurzen bandförmigen Körpers, sodass auch schwere Oberteile mit hoher Spannkraft zum Aufspringen vom Unterteil gebracht werden können, und aufgrund der Tatsache, dass der Springkörper als von den Behälterwänden materialverschiedenes Elastomerteil ausgebildet ist, ergeben sich neben einer hohen Federkraft noch zusätzlich eine lange Lebensdauer und eine überlegene Befestigungsart, weil sich der Springkörper mit zugeordneten Lappen direkt in das Oberteil und das Unterteil hinein erstreckt und dort an eine behälterinnenseitige Verbindungsbahn angebunden ist.

Durch die Ausbildung des Springkörpers aus einem weich-elastischen Material, das im 2k-Spritzgussverfahren an die aus einem härteren Material bestehenden Behälterwände angespritzt ist, ergibt sich der Vorteil einer hohen Spannkraft bei langer Lebensdauer, ohne dass Einschränkungen im Hinblick auf die Gebrauchstauglichkeit bezüglich der aus einem harten Material bestehenden Behälterwände gemacht werden müssen.

Durch die Fortsetzung des profilierten Springkörpers in Form von einstückig daran anschliessenden Lappen, die werkstoffeinstückig ein Teil mit dem Springkörper bilden, ergibt sich eine besonders große Spannlänge für den Springkörper, wie es bisher noch nicht bekannt war.

In einer Weiterbildung der Erfindung ist es im Übrigen vorgesehen, dass der Springkörper zusammen mit dem werkstoffeinstückig daran ansetzenden Lappen als 2K-Spritzgussteil ausgebildet ist, das heißt, er wird in die in materialhärteren Schalen des Ober- und Unterteils direkt eingespritzt.

Von besonderem Vorteil ist dabei auch, dass sich der Springkörper mit den werkstoffeinstückig daran ansetzenden Lappen in das Lamellenpaket fortsetzt, was bedeutet, dass auch die Lamellen des Lamellenpaketes aus dem gleichen werkstoffeinstückigen Material, wie der Springkörper und die an ihm ansetzenden Lappen, gebildet ist.

Damit ergibt sich der Vorteil, dass mit einem einzigen Spritzgussvorgang, der gesamte elastomere Springkörper, der aus einem weich-elastischen Material besteht, mit dem härter-elastischen Material der Schalen aus Ober- und Unterteil verbunden werden kann und auch bei einer Vielzahl von Lastspielen keine Bruchgefahr mehr besteht.

Von besonderem Vorteil ist die Ausbildung des Springkörpers auch im Hinblick darauf, dass eine geschlossene Schutzkappe unter Federvorspannung des Springkörpers für viele Monate in dieser gespannten Stellung verbleiben muss und die Spannkraft nicht verloren geht.

Durch die besondere Ausbildung des profilierten, etwa wellenförmigen Springkörpers mit seiner direkten Anbindung an die wandseitigen Stirnseiten von Ober- und Unterteil ergeben sich einerseits eine hohe Federkraft und andererseits eine hohe Lebensdauer mit Beibehaltung der Federkraft über einen langen Zeitraum, weil eine sehr Spannlänge für den Springkörper gewährleistet ist.

Im Folgenden wird die Erfindung anhand von lediglich einen Ausführungsweg darstellenden Zeichnungen näher erläutert. Hierbei gehen aus den Zeichnungen und ihrer Beschreibung weitere erfindungswesentliche Merkmale und Vorteile der Erfindung hervor.
Fig. 1: Perspektivische Ansicht einer Schutzkappe nach der Erfindung von der Öffnungsseite her
Fig. 2: Die Draufsicht auf die Anordnung nach Fig. 1 in Richtung des Pfeiles II in Fig. 1
Fig. 3: Die Stirnansicht der Schutzkappe nach Fig. 1 und 2 in Richtung des Pfeiles III in Fig. 2
Fig. 4: Die Seitenansicht der Schutzkappe in Richtung des Pfeiles IV in Fig. 2
Fig. 5: Die Draufsicht auf die geöffnete Schutzkappe auf die Innenseite
Fig. 6: Eine gegenüber Fig. 5 abgewandelte perspektivische Darstellung
Fig. 7: Die Draufsicht der geöffneten Schutzkappe von der Außenseite her
Fig. 8: Eine schematisierte Schnittansicht durch die Gelenkanordnung nach der Erfindung mit zwei aneinander gegenüberliegenden zueinander fluchtenden Filmscharnieren und eine mittig dazwischen angeordneten Springkörper
Fig. 9: Die gegenüber Fig. 8 abgewandelte Darstellung mit einer seitlichen Stirnansicht der Gelenkanordnung
Fig. 10: Ein Mittenschnitt durch die Gelenkanordnung
Fig. 11: Perspektivische Darstellung eines Teils des Springkörpers
Fig. 12: Eine gegenüber Fig. 11 abgewandelte Ausführungsform eines Springkörpers
Fig. 13: Eine gegenüber Fig. 11 und 12 abgewandelte Ausführungsform eines Springkörpers mit Darstellung der Anbindung an die werkstoffeinstückig daran anschließenden Lappen

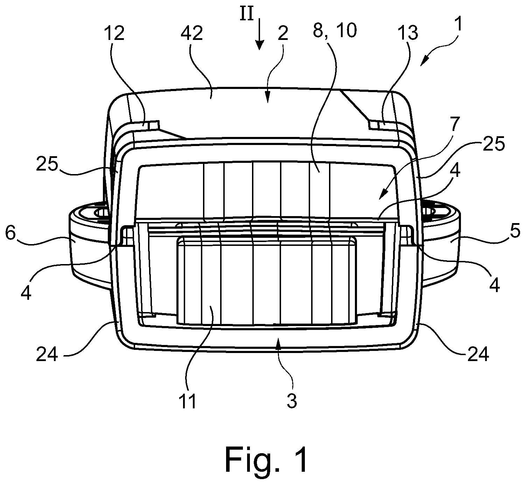

In den Fig. 1 - 3 ist eine Schutzkappe 1 dargestellt, die aus einem schalenförmigen Oberteil 2 besteht, das über eine Trennfuge 4 auf einem Unterteil 3 formschlüssig aufrastbar ist. Der Rastverschluss wird über zwei einander gegenüberliegende Bedienungshandhabung 5, 6 betätigt, wobei die Bedienungshandhaben 5, 6 in den jeweiligen Seitenwänden 24 des Unterteils 3 angeordnet sind.

Das schalenförmige Oberteil 2 bildet eine Deckfläche 42, die in abgerundeter Form in zwei einander gegenüberliegende Seitenwände 25 übergeht.

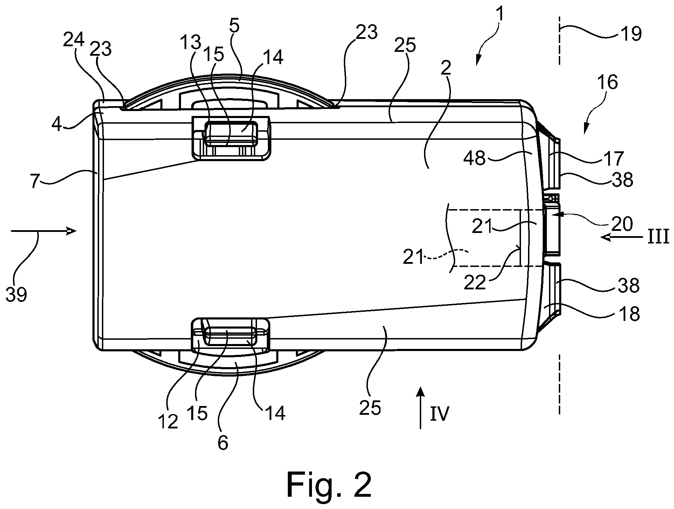

Gemäß Fig. 1 und 2 bildet die Schutzkappe 1 auf der einen Seite eine Öffnungsseite 7, in welche in Pfeilrichtung 39 ein zu schützendes Werkzeug oder die Spitze eines medizinischen Instrumentes in den Innenraum der einander zugeordneten schalenförmigen Ober- und Unterteile 2, 3 einschiebbar ist.

Die Einschuböffnung der Öffnungsseite 7 wird durch einander gegenüberliegende Lamellenpakete 10, 11 begrenzt, die eine Klemmöffnung bilden, sodass der dort einzuschiebende Gegenstand durch Klemmkraft der Haltelamellen 8, 9 der jeweiligen Lamellenpakete 10,11 kraftschlüssig gehalten ist.

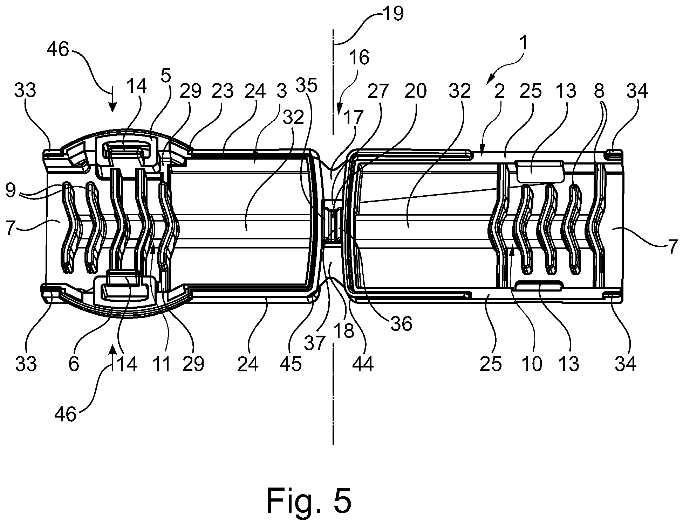

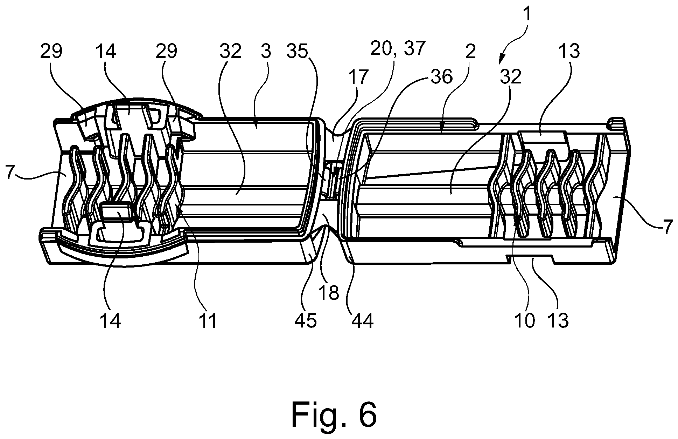

Die Fig. 5 und 6 zeigen als Beispiel eine Anzahl von parallel und im gegenseitigen Abstand zueinander angeordneten, konturierten elastomeren Haltelamellen 8, 9, die im gezeigten Ausführungsbeispiel zueinander fluchten. Ebenso können die Haltelamellen 8 des Oberteils 2 auf Lücke versetzt zu den Haltelamellen 9 des Unterteils 3 angeordnet sein, sodass eine stärkere Klemmwirkung auf einen in die Öffnungsseite 7 eingesteckten Gegenstand dann erreicht wird, wenn sie verzahnt ineinander eingreifen.

Ein besonderer Vorteil ist - wie in der allgemeinen Beschreibung angegeben, dass der später noch zu beschreibende Springkörper 20, der die Springkraft auf die Gelenkanordnung 16 ausübt, sich in Form von zwei elastomeren Verbindungsbahnen in das Material der Lamellen 8, 9 fortsetzt, sodass in einer bevorzugten Ausgestaltung der Erfindung es vorgesehen ist, dass der Springkörper 20 werkstoffeinstückig (materialeinstückig) mit den Verbindungsbahnen 32 und den auf der Innenseite der schalenförmigen Ober- und Unterteile 2, 3 angespritzten Haltelamellen 8, 9 verbunden ist.

Die Erfindung nicht auf diese Ausführung beschränkt. Es kann auch vorgesehen sein, dass die Verbindungsbahnen 32 vollkommen entfallen und dass die Haltelamellen 8, 9 aus einem völlig anderen Material bestehen als beispielsweise der Springkörper 20.

Vorteil der in den Fig. 5 und 6 dargestellten Ausführungsformen ist jedoch, dass sowohl der Springkörper 20 als auch die mit dem Springkörper werkstoffeinstückig verbundenen Lappen 21 und die mit den Lappen werkstoffeinstückig verbundenen Verbindungsbahnen 32 und diese wiederum mit den werkstoffeinstückigen Haltelamellen 8, 9 ein einziges elastomeres Teil aus einem weich-elastischen Kunststoff bilden.

Das gesamte Teil wird im Spritzgussverfahren an der Innenseite der Schalenteile angespritzt.

Neben den bogenförmigen Konturen der Haltelamellen 8, 9 können selbstverständlich alle beliebigen Formgebungen von Lamellen verwendet werden. Diese können auch als Druckpolster ausgebildet sein, das heißt, sie sind direkt aus einem elastomeren Material polsterförmig an der Innenseite jeweils des Ober- und Unterteils 2, 3 angespritzt.

Derartige Lamellen können auch als punktförmige Haltepunkte oder Klemmpunkte ausbildet sein und sie können auch aus einem harten Material bestehen, wodurch dann eine in der Formgebung genau definierte Öffnungsseite 7 für ein in der Formgebung dort einzusteckendes Instrument oder Werkzeug entsteht.

Die Verschlussanordnung besteht aus zwei einander gegenüberliegenden Bedienungshandhaben 5, 6, die genau symmetrisch zueinander angeordnet sind. Die Bedienungshandhaben 5 sind etwa bogenförmig ausgebildet und über seitliche Anbindungen 29 direkt an der jeweiligen Seitenwand 24 des Unterteils 3 spritzgusstechnisch angebunden.

Durch die elastomere Verformbarkeit einer Anbindung 29 an den Seitenwänden 24 ergibt sich der Vorteil, dass bei Druck auf die Bedienungshandhaben 5, 6 in Pfeilrichtung 46 die Anbindungen 29 elastisch nachgeben und somit die in diesem Bereich angeordnete Rastklinke 14 nach innen bewegen.

Jeder Bedienungshandhabe 5, 6 ist eine Rastklinke 14 zugeordnet und jede Rastklinke 14 greift in eine zugeordnete Rastausnehmung 13 im Bereich des Oberteils 2 ein.

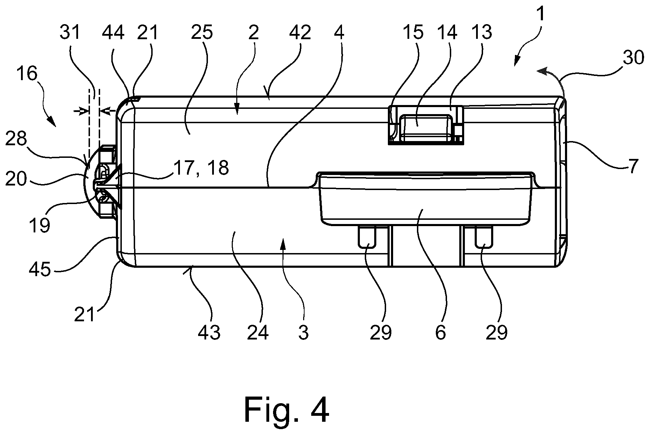

Gemäß Fig. 4 übergreift die Rastklinke 14 eine gehäusefeste Rastkante im Oberteil 2, um so eine Verrastung zwischen dem Oberteil 2 und dem Unterteil 3 zu erreichen.

Sobald ein Druck in Pfeilrichtung 46 auf die Bedienungshandhaben 5, 6 mit den Fingern einer Hand ausgeübt wird, werden die Rastklinken gegeneinander, unter Verringerung des gegenseitigen Abstandes, bewegt und geraten außer Eingriff mit den Rastausnehmungen 12, 13 im Oberteil 2.

Die Seitenflächen der jeweiligen Bedienungshandhaben 5, 6 setzen über Verbindungsstellen 23 an den Seitenwänden 24 des Oberteils 2 an.

Die Verbindungsstelle 23 in Verbindung mit der elastomeren Anbindung 29 bildet das elastomere Rückstellmoment für die Bedienungshandhaben 5, 6, die somit in Gegenrichtung zur Pfeilrichtung 46 jeweils federbelastet sind.

Von besonderem Vorteil ist auch die Ausbildung eines Filmscharniers und einem mittig dazwischen angeordneten Springkörper mit hoher Federkraft.

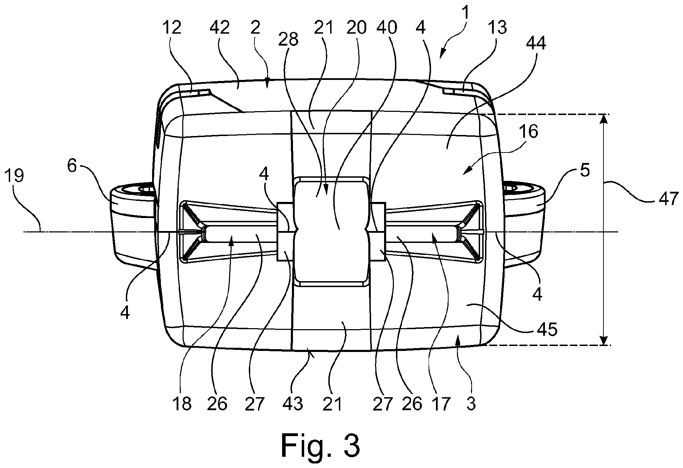

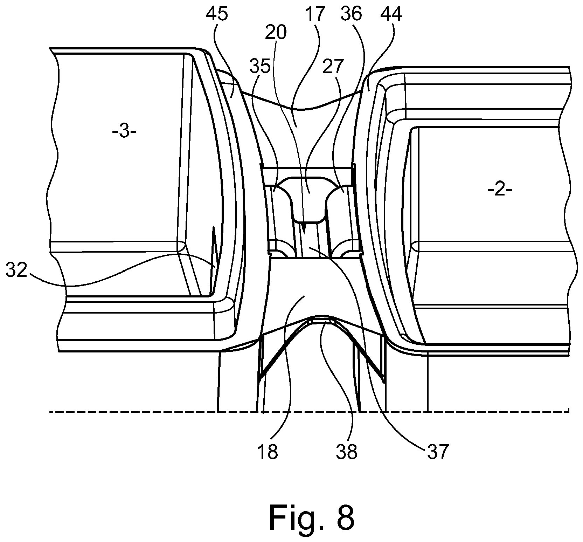

Gemäß Fig. 3 werden zur Überbrückung der jeweiligen Stirnseite 44 des Oberteils 2 zur Stirnseite 45 des Unterteils 3 jeweils ein Filmscharnier 17, 18 verwendet, das gemäß Fig. 8 etwa bogenförmig profiliert ist und eine mittlere Biegelinie 38 ausbildet.

Auf diese Weise ist gewährleistet, dass eine Verschwenkung der Gelenkanordnung 16 entlang der Schwenkachse 19 zwischen den einander zugeordneten Stirnseiten 44, 45 von Oberteil 2 und Unterteil 3 stattfindet.

Wichtig ist gemäß Fig. 4, dass bei Entsperrung der Rastvorrichtung das Oberteil 2 in Pfeilrichtung 30 unter der Federwirkung des Springkörpers 20 selbsttätig aufspringt und in dieser Stellung verharrt.

Aus Fig. 4 wird deutlich, dass der Springkörper 20 als massiver - von der üblichen Bandstruktur abweichender - Körper außerhalb der Schwenkachse 19 der Filmscharniere 17 angeordnet ist und diese Schwenkachse überbrückt.

Über die Wahl des Abstandes 31 wird das Öffnungsmoment in Pfeilrichtung 30 definiert.

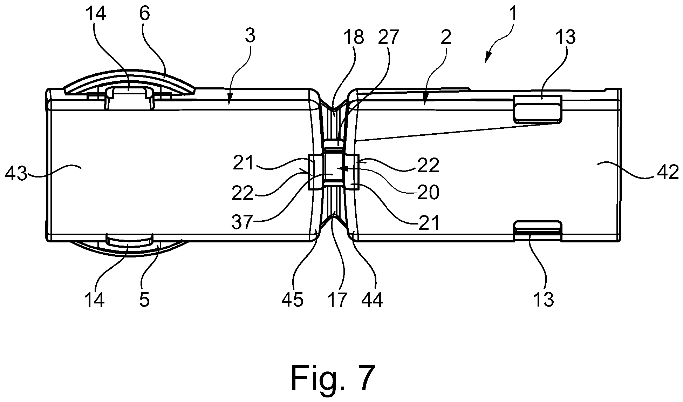

Eine besonders lange Federlänge ergibt sich erfindungsgemäß dadurch, dass sich der Springkörper 20 gemäß Fig. 7 werkstoffeinstückig in Lappen 21 fortsetzt, welche Lappen 21 jeweils an der Deckfläche 42, 43 des Oberteils angreifen und mit zugeordnete Übergangskanten 22 an den Flächen 42, 43 von Oberteil und Unterteil 2, 3 angebunden sind.

Damit ergibt sich eine besonders lange Federlänge des Springkörpers 20, was bisher im Stand der Technik nicht bekannt war.

Von besonderem Vorteil ist weiterhin, dass sich die jeweiligen Lappen 21, die werkstoffeinstückig mit dem Material des Springkörpers 20 verbunden sind, nach innen an der Innenseite der schalenförmigen Ober- und Unterteile 2, 3 in der Art von Verbindungsbahnen 32 fortsetzen und einen werkstoffeinstückigen Verbund mit den an den Verbindungsbahnen 32 einstückig angespritzten Lamellenpaketen 10, 11 bilden.

Von besonderem Vorteil ist, dass im mittigen Bereich zwischen den einander in gleicher Flucht angeordneten Filmscharnieren 17, 18 nunmehr ein profilierter Springkörper 20 angeordnet ist, der im bevorzugten Ausführungsbeispiel (s. Fig. 6, Fig. 7, Fig. 5, Fig. 8 und Fig. 11) etwa bogen- oder wellenförmig profiliert ist.

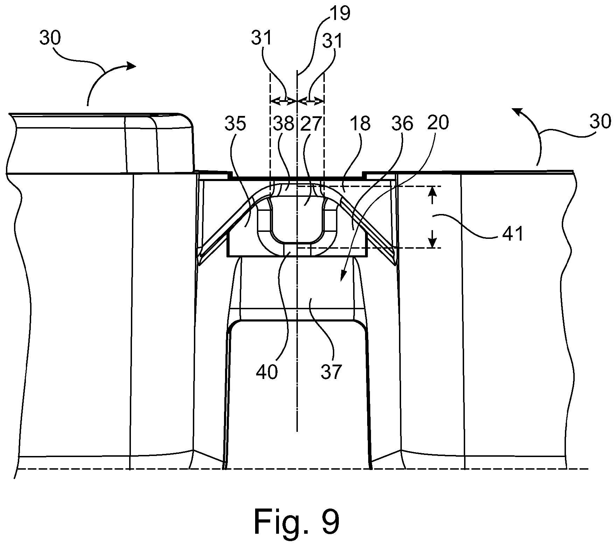

Die Stirnseiten 26 der Filmscharniere bilden somit Biegelinien 38 und der dazwischen angeordnete Springkörper 20 bildet eine Federkante 40, die am besten in Fig. 9 erkennbar ist.

Der Springkörper 20 ist spiegelsymmetrisch profiliert und besteht aus zwei einander gegenüberliegenden Bogenstrukturen 35, 36, zwischen denen werkstoffeinstückig ein Mittenschenkel 37 angeordnet ist.

Die Bogenstrukturen 35, 36 setzen sich werkstoffeinstückig in die Lappen 21 fort und diese setzen an den einander gegenüberliegenden Stirnseiten 44, 45 von Ober- und Unterteil 2, 3 an.

Damit ergibt sich eine besonders große Federlänge, die als Federlänge 47 in Fig. 3 eingetragen ist.

Der profilierte Springkörper 20 ist im Bereich einer mittigen Freistellung 27 zwischen den einander auf gleicher Flucht befindlichen nebeneinanderliegend angeordneten Filmscharnieren 17, 18 angeordnet.

Der profilierte Springkörper 20 bildet somit eine Bogenfläche 28 (s. Fig. 3 und Fig. 4), die eine mittige Federkante 40 definiert über die sich der Springkörper 20 elastomer verformt.

Somit ergeben sich von der mittigen Federkante 40 des Mittenschenkels 37 des wellenförmig profilierten Springkörpers 20 Abstände 31, die jeweils links und rechts von der Schwenkachse 19 ausgebildet sind, wobei die Abstände 31 den Abstand zwischen der Federkante 40 und den Bogenstrukturen 35, 36 des Springkörpers 20 beschreiben.

Der Abstand 41 beschreibt die Profiltiefe des wellenförmig profilierten Springkörpers 20 und der Abstand 41 beeinflusst das Aufspringmoment zwischen Oberteil 2 und Unterteil 3.

Die besondere Bogenstruktur des Springkörpers 20 ist insbesondere aus den Fig. 8 und 9 zu entnehmen. Die Bogenstrukturen 35, 36 erhöhen das Aufspringmoment des Springkörpers 20 in wesentlichem Maß.

Der Abstand 41 definiert die Tiefe der Wellenstruktur, die sich zwischen den seitlichen Bogenstrukturen 35, 36 und dem Mittenschenkel 37 ergibt.

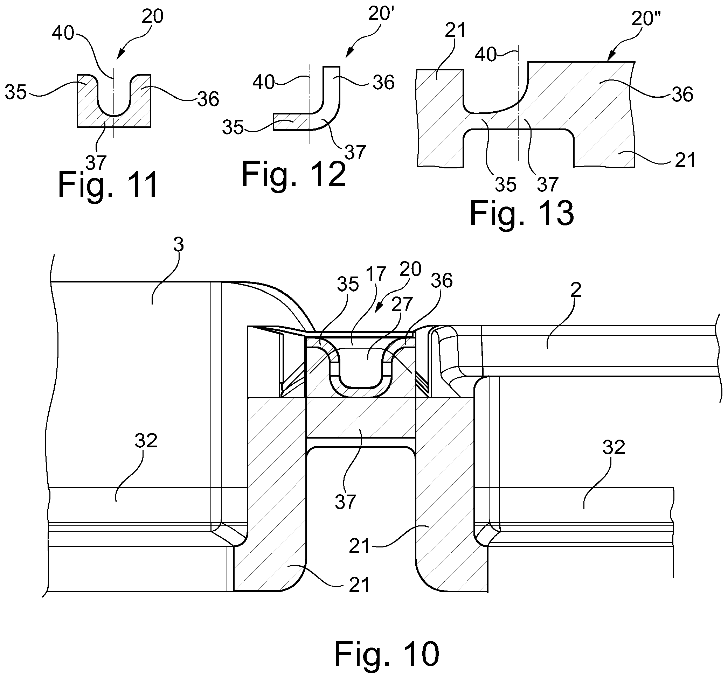

Die Fig. 11 zeigt schematisiert eine derartige Bogenstruktur, ohne die seitlichen Anbindungen über die Lappen 21 und dergleichen zu zeigen. Für die grundsätzliche Verwirklichung der Erfindung reicht aus, dass der Springkörper wellenförmig ausgebildet ist, ohne dass die Fortsetzung in Form von Lappen notwendig ist.

Die Sprungkraft wird jedoch durch die Fortsetzung des Springkörpers in die Lappen gesteigert.

Die Fig. 12 zeigt ein abgewandeltes Ausführungsbeispiel eines profilierten Springkörpers 20, wo erkennbar ist, dass das Profil des Springkörpers 20 auch als einfaches L-Profil ausgebildet sein kann, wobei dann die beiden Bogenstrukturen 35, 36 nicht mehr spiegelsymmetrisch zueinander liegen. Der Mittenschenkel 37 ist auch in Bezug zur Federkante 40 versetzt.

Ein ähnliches Ausführungsbeispiel zeigt auch die Fig. 13 im Vergleich zu Fig. 12, wo erkennbar ist, dass es möglich ist, den Mittenschenkel relativ dick auszubilden und direkt in eine großprofilierte rechte Bogenstruktur 36 zu überführen, während die linke Bogenstruktur 35 kleiner ausgebildet ist.

Auch hier erfolgt die direkte Anbindung an die werkstoffeinstückig daran anschließenden Lappen 21, sodass damit ein etwas anders profilierter Springkörper 20', gemäß Fig. 12 oder 20" gemäß Fig. 13 dargestellt ist.

Um eine Zentrierung zwischen dem Oberteil 2 und dem Unterteil 3 vorzusehen, ist gemäß Fig. 5 vorgesehen, dass an der Innenseite des Oberteils 2 Zentrieraussparungen 34 vorgesehen sind, die mit zugeordneten Zentriernasen 33 am Unterteil 3 in Eingriff kommen, wenn die beiden Teile 2, 3 miteinander verrastet sind.

### Zeichnungslegende:

- 1:: Schutzkappe
- 2:: Oberteil
- 3:: Unterteil
- 4:: Trennfuge
- 5:: Bedienungshandhabe
- 6:: Bedienungshandhabe
- 7:: Öffnungsseite
- 8:: Haltelamelle (oben)
- 9:: Haltelamelle (unten)
- 10:: Lamellenpaket (oben)
- 11:: Lamellenpaket (unten)
- 12:: Rastausnehmung
- 13:: Rastausnehmung
- 14:: Rastklinke
- 15:: Rastkante
- 16:: Gelenkanordnung
- 17:: Filmscharnier
- 18:: Filmscharnier
- 19:: Schwenkachse
- 20:: Springkörper 20', 20"
- 21:: Lappen (von 21)
- 22:: Übergangskante
- 23:: Verbindungsstelle
- 24:: Seitenwand (Unterteil 3)
- 25:: Seitenwand (Oberteil 2)
- 26:: Stirnseite (von 17, 18)
- 27:: Freistellung
- 28:: Bogenfläche (von 20)
- 29:: Anbindung
- 30:: Pfeilrichtung
- 31:: Abstand (zw. 19 + 20)
- 32:: Verbindungsbahn
- 33:: Zentriernase
- 34:: Zentrieraussparung
- 35:: Bogenstruktur (von 20)
- 36:: Bogenstruktur (von 20)
- 37:: Mittenschenkel (von 20)
- 38:: Biegelinie (von 17, 18)
- 39:: Pfeilrichtung
- 40:: Federkante
- 41:: Abstand (zw. 38 + 37)
- 42:: Deckfläche (von 2)
- 43:: Deckfläche (von 3)
- 44:: Stirnseite (von 2)
- 45:: Stirnseite (von 3)
- 46:: Pfeilrichtung
- 47:: Federlänge

## Patentansprüche

1. Schutzkappe (1) mit Springöffnung, bestehend aus einem Ober- und einem Unterteil (2, 3), die über Filmscharniere (17, 18) einseitig schwenkbar miteinander verbunden und im Schließzustand miteinander verrastet sind, wobei das Oberteil (2) unter der Federlast eines profilierten Springkörpers (20, 20', 20") in Öffnungsrichtung gegenüber dem Unterteil (3) vorgespannt ist, sodass bei Betätigung einer Rastvorrichtung (12-15) das Oberteil (2) vom Unterteil (3) unter der Kraft eines Kraftspeichers aufschwenkt und in der geöffneten Stellung verbleibt, wobei der profilierte Springkörper (20, 20', 20") aus einem weich-elastischen elastomeren Kunststoffmaterial besteht und mit werkstoffeinstückig daran ansetzenden, endseitigen Lappen (21) an Stirnseiten (44, 45) von Ober- und Unterteil (2, 3) befestigt ist, **dadurch gekennzeichnet, dass** die Schutzkappe (1) in ihrem Innenraum einander gegenüberliegende Lamellenpakete (10, 11) oder elastomere Druckpolster aufweist, wobei das eine Lamellenpaket (10) oder elastomere Druckpolster an der Innenseite des Oberteils (2) und das gegenüberliegende Lamellenpaket (11) oder elastomere Druckpolster an der Innenseite des Unterteils (3) angeordnet ist, und die beiden Lamellenpakete (10, 11) oder elastomeren Druckpolster zwischen sich eine Klemmaufnahme bilden, die einen dort eingeschobenen, zu schützenden Gegenstand gegen ein Herausziehen schützt.

2. Schutzkappe (1) nach Anspruch 1, **dadurch gekennzeichnet, dass** sich der Springkörper (20, 20', 20") mit den werkstoffeinstückig angeformten endseitigen Lappen (21) in einer elastomeren Verbindungsbahn (32) in die Innenseite von Ober- und Unterteil (2, 3) fortsetzt und dort angespritzt ist.

3. Schutzkappe (1) nach Anspruch 2, **dadurch gekennzeichnet, dass** die elastomere Verbindungsbahn (32) werkstoffeinstückig mit den Lamellenpaketen (10, 11) verbunden ist, welche an den Innenseiten von Ober- und Unterteil (2, 3) angespritzt sind.

4. Schutzkappe (1) nach Anspruch 2, **dadurch gekennzeichnet, dass** die elastomere Verbindungsbahn (32) werkstoffeinstückig mit den Druckpolstern verbunden ist, welche an den Innenseiten von Ober- und Unterteil (2, 3) angespritzt sind.

5. Schutzkappe (1) mit Springöffnung nach einem der Ansprüche 1 bis 4, **dadurch gekennzeichnet, dass** die Rastvorrichtung (12-15) zwischen dem Ober- und dem Unterteil an Seitenwänden (24, 25) des Ober- und Unterteils (2, 3) angeordnet ist.

6. Schutzkappe (1) nach Anspruch 5, **dadurch gekennzeichnet, dass** die Rastvorrichtung (12-15) mit seitlichen, gegenüberliegenden Bedienungshandhaben (5, 6) betätigbar ist.

7. Schutzkappe (1) nach Anspruch 6, **dadurch gekennzeichnet, dass** die Bedienungshandhaben (5, 6) über seitliche Anbindungen (29) direkt an der jeweiligen Seitenwand (24) des Unterteils (3) spritzgusstechnisch angebunden sind.

8. Schutzkappe (1) nach einem der Ansprüche 6 oder 7, **dadurch gekennzeichnet, dass** jeder Bedienungshandhabe (5, 6) eine Rastklinke (14) zugeordnet ist, und dass jede Rastklinke (14) in eine zugeordnete Rastausnehmung (13) im Bereich des Oberteils (2) eingreift.

9. Schutzkappe (1) nach einem der Ansprüche 1 bis 8, **dadurch gekennzeichnet, dass** der Springkörper (20) als U-Profil ausgebildet ist oder eine Wellenform aufweist.

10. Schutzkappe (1) nach einem der Anspruch 1 bis 8, **dadurch gekennzeichnet, dass** der Springkörper (20', 20") als Dreiecks-Profil ausgebildet ist.

## Claims

1. Protective cap (1) with resilient opening consisting of an upper part and a lower part (2, 3) which are connected to one another to be pivotable on one side via integral hinges (17, 18) and locked to one another in the closed state, wherein the upper part (2) is pretensioned under the spring load of a profiled resilient body (20, 20', 20") in the opening direction with respect to the lower part (3) so that on actuating a latch device (12-15), the upper part (2) pivots upwards from the bottom part (3) under the force of a force store and remains in the opened position, wherein the profiled resilient body (20, 20', 20") consists of a soft-elastic elastomeric plastic material and is attached to end-face sides (44, 45) of top part and bottom part (2, 3) by end-side flaps (21) attaching thereto from the same material, **characterised in that** the protective cap (1) has rib packages (10, 11) or elastomeric pressure cushions opposite one another in its interior, wherein the one rib package (10) or elastomeric pressure cushions is arranged on the inner side of the upper part (2) and the opposite rib package (11) or elastomeric pressure cushions on the inner side of the lower part (3), and the two rib packages (10, 11) or elastomeric pressure cushions form between them a clamping receiver which protects an object inserted there and to be protected against withdrawal.

2. Protective cap (1) according to claim 1, **characterised in that** the resilient body (20, 20', 20") with the end-side flaps (21) integrally moulded from the same material in an elastomeric connecting web (32) continues into the inner side of upper part and lower part (2, 3) and is integrally injection-moulded there.

3. Protective cap (1) according to claim 2, **characterised in that** the elastomeric connecting web (32) from the same material is connected to the rib packages (10, 11) which are integrally injection-moulded on the inner sides of upper part and lower part (2, 3).

4. Protective cap (1) according to claim 2, **characterised in that** the elastomeric connecting web (32) from the same material is connected to the pressure cushions which are integrally injection-moulded on the inner sides of upper part and lower part (2, 3).

5. Protective cap (1) with resilient opening according to one of claims 1 to 4, **characterised in that** the latch device (12-15) is arranged between the upper part and the lower part on side walls (24, 25) of the upper part and lower part (2, 3).

6. Protective cap (1) according to claim 5, **characterised in that** the latch device (12-15) can be actuated by lateral, opposite operating grips (5, 6).

7. Protective cap (1) according to claim 6, **characterised in that** the operating grips (5, 6) are connected using injection-moulding technology directly to the respective side wall (24) of the lower part (3) via lateral connections (29).

8. Protective cap (1) according to one of claims 6 or 7, **characterised in that** a latch (14) is assigned to each operating grip (5, 6), and **in that** each latch (14) engages in an assigned latch recess (13) in the region of the upper part (2).

9. Protective cap (1) according to one of claims 1 to 8, **characterised in that** the resilient body (20) is formed as a U profile or has a wave shape.

10. Protective cap (1) according to one of claims 1 to 8, **characterised in that** the resilient body (20', 20") is formed as a triangular profile.

## Revendications

1. Cache de protection (1) avec ouverture à ressort, consistant en une pièce supérieure et une pièce inférieure (2, 3) qui sont reliées l'une à l'autre de manière pivotante d'un côté par des charnières à film (17, 18) et sont en prise mutuelle à l'état fermé, dans lequel la pièce supérieure (2) est contrainte par rapport à la pièce inférieure (3) sous la charge élastique d'un corps de ressort profilé (20, 20', 20") dans la direction d'ouverture, de sorte que lors de l'actionnement d'un dispositif d'enclenchement (12-15), la pièce supérieure (2) s'ouvre par pivotement depuis la pièce inférieure (3) sous la force d'un accumulateur de force et reste dans la position ouverte, dans lequel le corps de ressort profilé (20, 20', 20") est en un matériau synthétique élastomère et mollement élastique et est fixé aux côtés frontaux (44, 45) des pièces supérieure et inférieure (2, 3) avec des pattes côté extrémité (21) rapportées sur lui, venues de matière, **caractérisé en ce que** le cache de protection (1) comporte dans son espace intérieur des paquets de lamelles (10, 11) ou tampons de pression élastomères se faisant mutuellement face, dans lequel un paquet de lamelles (10) ou tampon de pression élastomère est disposé du côté intérieur de la pièce supérieure (2) et le paquet de lamelles (11) ou tampon de pression élastomère opposé est disposé du côté intérieur de la pièce inférieure (3), et les deux paquets de lamelles (10, 11) ou tampons de pression élastomères forment entre eux un logement de serrage qui protège un objet à protéger glissé à cet endroit d'une extraction.

2. Cache de protection (1) selon la revendication 1, **caractérisé en ce que** le corps de ressort (20, 20', 20") avec les pattes côté extrémité (21) rapportées venues de matière se prolonge dans une bande de liaison élastomère (32) du côté intérieur des pièces supérieure et inférieure (2, 3) et est rapporté par injection à cet endroit.

3. Cache de protection (1) selon la revendication 2, **caractérisé en ce que** la bande de liaison élastomère (32) est reliée d'un seul tenant aux paquets de lamelles (10, 11) qui sont rapportés par injection sur les côtés intérieurs des pièces supérieure et inférieure (2, 3).

4. Cache de protection (1) selon la revendication 2, **caractérisé en ce que** la bande de liaison élastomère (32) est reliée d'un seul tenant aux tampons de pression qui sont rapportés par injection sur les côtés intérieurs des pièces supérieure et inférieure (2, 3).

5. Cache de protection (1) à ouverture à ressort selon l'une des revendications 1 à 4, **caractérisé en ce que** le dispositif d'enclenchement (12-15) est disposé entre les pièces supérieure et inférieure sur des parois latérales (24, 25) des pièces supérieure et inférieure (2, 3).

6. Cache de protection (1) selon la revendication 5, **caractérisé en ce que** le dispositif d'enclenchement (12-15) est apte à être actionné avec des poignées de manipulation (5, 6) latérales opposées.

7. Cache de protection (1) selon la revendication 6, **caractérisé en ce que** les poignées de manipulation (5, 6) sont attachées par des attaches latérales (29) directement à la paroi latérale (24) respective de la pièce inférieure (3) avec une technique de moulage par injection.

8. Cache de protection (1) selon l'une des revendications 6 ou 7, **caractérisé en ce qu'à** chaque poignée de manipulation (5, 6) est associé un cran d'enclenchement (14) et que chaque cran d'enclenchement (14) vient en prise dans un creux d'enclenchement (13) associé dans la zone de la pièce supérieure (2).

9. Cache de protection (1) selon l'une des revendications 1 à 8, **caractérisé en ce que** le corps de ressort (20) est conformé en un profilé en U ou présente une forme ondulée.

10. Cache de protection (1) selon l'une des revendications 1 à 8, **caractérisé en ce que** le corps de ressort (20', 20") est conformé en un profilé triangulaire.
